(19) **Europäisches Patentamt**
**European Patent Office**
**Office européen des brevets**

(11) **EP 1 392 255 B1**

(12) **EUROPÄISCHE PATENTSCHRIFT**

(45) Veröffentlichungstag und Bekanntmachung des Hinweises auf die Patenterteilung:
**08.06.2011  Patentblatt 2011/23**

(21) Anmeldenummer: **02738009.6**

(22) Anmeldetag: **30.04.2002**

(51) Int Cl.:
*A61K 9/51* (2006.01)          *A61K 47/42* (2006.01)
*A61K 47/48* (2006.01)         *A61P 25/04* (2006.01)
*A61P 25/28* (2006.01)         *A61K 38/08* (2006.01)
*A61K 31/451* (2006.01)        *A61K 31/4741* (2006.01)
*A61K 31/704* (2006.01)

(86) Internationale Anmeldenummer:
**PCT/EP2002/004735**

(87) Internationale Veröffentlichungsnummer:
**WO 2002/089776 (14.11.2002 Gazette 2002/46)**

(54) **NANOPARTIKEL AUS PROTEIN MIT GEKOPPELTEM APOLIPOPROTEIN E ZUR ÜBERWINDUNG DER BLUT-HIRN-SCHRANKE UND VERFAHREN ZU IHRER HERSTELLUNG**

NANOPARTICLES MADE OF PROTEIN WITH COUPLED APOLIPOPROTEIN E FOR PENETRATION OF THE BLOOD-BRAIN BARRIER AND METHODS FOR THE PRODUCTION THEREOF

NANOPARTICULES CONSTITUEES D'UNE PROTEINE A APOLIPOPROTEINE E COUPLEE ET SERVANT A TRAVERSER LA BARRIERE SANG-CERVEAU ET PROCEDES POUR LEUR PRODUCTION

(84) Benannte Vertragsstaaten:
**AT BE CH CY DE DK ES FI FR GB GR IE IT LI LU MC NL PT SE TR**

(30) Priorität: **05.05.2001  DE 10121982**

(43) Veröffentlichungstag der Anmeldung:
**03.03.2004  Patentblatt 2004/10**

(73) Patentinhaber: **LTS LOHMANN Therapie-Systeme AG**
**56626 Andernach (DE)**

(72) Erfinder:
• **KREUTER, Jörg**
**61350 Bad Homburg (DE)**
• **LANGER, Klaus**
**61137 Schöneck (DE)**
• **WEBER, Carolin**
**93057 Regensburg (DE)**
• **ALYAUTDIN, Renad, N.**
**Moscow, 123007 (RU)**

(74) Vertreter: **Flaccus, Rolf-Dieter**
**Flaccus · Müller-Wolff**
**Patentanwälte**
**Bussardweg 10**
**50389 Wesseling (DE)**

(56) Entgegenhaltungen:
**EP-A2- 1 300 139**

• **LANGER K ET AL: "Preparation of avidin-labeled protein nanoparticles as carriers for biotinylated peptide nucleic acid" EUROPEAN JOURNAL OF PHARMACEUTICS AND BIOPHARMACEUTICS, ELSEVIER SCIENCE PUBLISHERS B.V., AMSTERDAM, NL, Bd. 49, Nr. 3, 2. Mai 2000 (2000-05-02), Seiten 303-307, XP004257171 ISSN: 0939-6411**
• **KREUTER J ET AL: "INFLUENCE OF THE TYPE OF SURFACTANT ON THE ANALGESIC EFFECTS INDUCED BY THE PEPTIDE DALARGIN AFTER ITS DELIVERY ACROSS THE BLOOD-BRAIN BARRIER USING SURFACTANT-COATED NANOPARTICLES" JOURNAL OF CONTROLLED RELEASE, ELSEVIER SCIENCE PUBLISHERS B.V. AMSTERDAM, NL, Bd. 49, Nr. 1, 10. November 1997 (1997-11-10), Seiten 81-87, XP000667154 ISSN: 0168-3659**

**Beschreibung**

Die vorliegende Erfindung betrifft Nanopartikel aus einem hydrophilen Protein oder einer Kombination hydrophiler Proteine, vorzugsweise aus Serumalbumin, insbesondere humaner Herkunft, die mittels gebundenem Apolipoprotein E die Blut-Hirn-Schranke überwinden können, um pharmazeutisch oder biologisch aktive Wirkstoffe in den Liquor cerebrospinalis zu transportieren.

Nanopartikel sind Partikel mit einer Größe zwischen 10 und 1000 nm, die aus künstlichen oder natürlichen makromolekularen Substanzen hergestellt werden können. An derartige Nanopartikel können Arzneistoffe oder anderes biologisch aktives Material kovalent, ionisch oder adsorptiv gebunden oder in das Material der Nanopartikel inkorporiert werden.

Bisher waren jedoch nur Nanopartikel aus Polyalkylcyanoacrylaten, die mit Polysorbat 80 (Tween® 80) oder anderen Tensiden überzogen wurden, in der Lage, die Blut-Hirn-Schranke zu überwinden, um hydrophile Arzneistoffe zum Liquor cerebrospinalis zu transportieren und pharmakologische Effekte hervorzurufen. Der Mechanismus dieses Transports beruht nach bisherigen Untersuchungen darauf, dass Apolipoprotein E (ApoE) durch den Überzug aus Polysorbat 80 von den Nanopartikeln adsorbiert wird. Dadurch täuschen diese Partikel vermutlich Lipoprotein-Partikel vor, die von den LDL-Rezeptoren der Gehirnkapillar-Endothelzellen, welche die Lipidversorgung des Gehirns gewährleisten, erkannt und gebunden werden.

Eine Reihe von Arzneistoffen konnte mittels mit Polysorbat 80 oder anderen Tensiden überzogenen Polybutylcyanoacrylat-Nanopartikeln über die Blut-Hirn-Schranke transportiert werden und einen signifikanten pharmakologischen Effekt hervorrufen. Beispiele für derartig verabreichte Arzneimittel sind Dalargin, ein Endorphin-Hexapeptid, Loperamid und Tubocuarin, die beiden NMDA-Rezeptor-Antagonisten MRZ 2/576 bzw. MRZ 2/596 der Fa. Merz, Frankfurt, sowie das Antikrebsmittel Doxorubicin.

Die Nachteile der Polybutylcyanoacrylat-Manopartikel liegen jedoch-darin, dass Polysorbat 80 nicht physiologisch ist und dass der Transport über die Blut-Hirn-Schranke möglicherweise auf einem toxischen Effekt des Polysorbat 80 beruhen könnte. Ein Überzug von Polybutylcyanoacrylat-Nanopartikeln mit Polysorbat 80 oder anderen Tensiden ist jedoch essentiell für den Transport der Polybutylcyano-acrylat-Nanopartikel über die Blut-Hirn-Schranke. Die bekannten Polybutylcyanoacrylat-Nanopartikel haben jedoch den weiteren Nachteil, dass sowohl die Bindung des ApoEs wie auch der Arzneistoffe nur adsorptiv erfolgt. Dadurch liegt das an die Nanopartikel gebundene ApoE bzw. der Arzneistoff im Gleichgewicht mit freiem ApoE bzw. Arzneistoff vor, und es kann nach Injektion in den Organismus eine schnelle Desorption dieser Stoffe von den Partikeln erfolgen. Außerdem binden die meisten Arzneistoffe nicht in ausreichendem Maß an Polybutylcyanoacrylat-Nanopartikel und können somit nicht mit Hilfe dieses Trägersystems über die Blut-Hirn-Schranke transportiert werden.

Der vorliegenden Erfindung lag die Aufgabe zugrunde, Nanopartikel zur Überwindung der Blut-Hirn-Schranke bereitzustellen, welche die vorgenannten Nachteile nicht haben und die unter Vermeidung nicht physiologischer Tenside das für den Transport über die Blut-Hirn-Schranke notwendige Apolipoprotein E nicht lediglich adsorbiert haben.

Die Aufgabe wurde überraschenderweise durch Nanopartikel gelöst, die aus einem hydrophilen Protein oder einer Kombination hydrophiler Proteine, vorzugsweise aus Serumalbumin, besonders bevorzugt aus humanem Serumalbumin, oder einem vergleichbaren Protein bestehen, an die Apolipoprotein E kovalent oder über das Avidin/Biotin-System gekoppelt ist.

Bei den Albuminen handelt es sich um eine Gruppe von Proteinen, die vor allem in tierischen/menschlichen Flüssigkeiten, z.B. das Serumalbumin im Blut, oder Geweben vorkommen. Albumine sind reich an negativ geladenen Aminosäuren sowie an Leucin und Isoleucin. Im Vergleich zu den sie begleitenden Globulinen besitzen die Albumine niedrigere Molmassen und sind erst durch relativ hohe Salzkonzentrationen ausfällbar.

Auch Gelatine A, Gelatine B, Casein oder vergleichbare Proteine sind als Ausgangsproteine für die erfindungsgemäßen Nanopartikel geeignet.

Das Apolipoprotein E ist eine Komponente der Lipoprotein-Komplexe. Diese Komplexe aus Lipiden und Apolipoproteinen ermöglicht den Transport der im Wasser unlöslichen Lipide im Blut. Das ApoE vermittelt vermutlich den Transport der erfindungsgemäßen Nanopartikel über die Blut-Hirn-Schranke, indem es an die LDL-Rezeptoren der Gehirnkapillar-Endothelzellen bindet.

Die erfindungsgemäßen Nanopartikel können zusätzlich ein oder mehrere funktionelle Proteine aufweisen, die über bifunktionale Spacermoleküle an Thiolgruppen der mit Thiolgruppen modifizierten Nanopartikel gebunden sind. Zur Präparation derartiger Nanopartikel können die auf der Oberfläche der Nanopartikel befindlichen funktionellen Gruppen (Aminogruppen, Carboxylgruppen, Hydroxylgruppen) durch geeignete Reagenzien zu reaktiven Thiolgruppen umgesetzt werden. Funktionelle Proteine können dann über bifunktionale Spacermoleküle, die eine Reaktivität sowohl gegenüber Aminogruppen als auch freien Thiolgruppen aufweisen, an die Thiolgruppen-modifizierten Nanopartikel gebunden werden. Die auf diese Weise an die Nanopartikel zu koppelnden funktionellen Proteine können aus der Gruppe ausgewählt werden, die Avidin, Avidinderivate, Apolipoproteine, wie z. B. Apolipoprotein E, aber auch Antikörper, Enzyme und dergleichen umfaßt. Dabei können die funktionellen Proteine selbst eine pharmakologische oder biologische Wirkung haben.

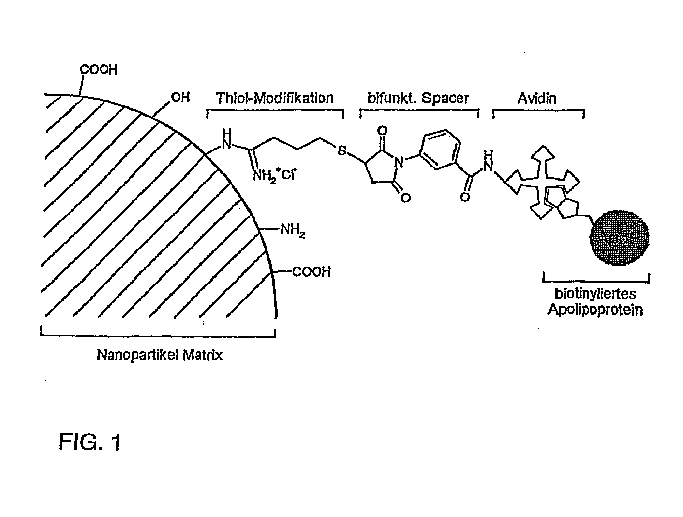

In einer bevorzugten Ausführungsform weisen die erfindungsgemäßen Nanopartikel kovalent gekoppeltes Avidin auf, über das biotinyliertes Apolipoprotein E gebunden werden kann, wie in Figur 1 veranschaulichend dargestellt ist. Avidin selbst ist ein für Biotin hochaffines Glykoprotein, das durch die zuvor erwähnen bifunktionalen Spacermoleküle kovalent an die Thiolgruppen, der thiolierten Nanopartikel gebunden sein kann. Durch die kovalente Bindung des Avidins an die Nanopartikel kann nicht nur biotinyliertes ApoE, das für den Transport über die Blut-Hirn-Schranke notwendig ist, gebunden werden, sondern es können eine Vielzahl von biotinylierten Molekülen besonders effizient von den Avidin-modifizierten Nanopartikeln gebunden werden. Besonders bevorzugt werden dabei pharmakologisch oder biologisch aktive Moleküle.

Um pharmakoloigsche Effekte zu vermitteln, können die erfindungsgemäßen Nanopartikel pharmakologisch oder biologisch aktive Substanzen aufweisen. Diese Wirkstoffe können in die Nanopartikel inkorporiert sein oder werden von den Nanopartikeln gebunden. Dabei kann die Bindung der pharmakologisch oder biologisch aktiven Wirkstoffe sowohl kovalent, komplexierend über das Avidin-Biotin-System als auch inkorporativ oder adsorptiv erfolgen.

Die erfindungsgemäßen Nanopartikel eignen sich besonders gut, um Arzneistoffe, die keinen oder keinen ausreichenden Übergang über die Blut-Hirn-Schranke aufweisen, wie beispielsweise Dalargin, Loperamid, Tubocuarin oder Doxorubicin oder dergleichen, zu binden und über die Blut-Hirn-Schranke zu transportieren und pharmakologische Effekte hervorzurufen.

Das Verfahren zur Herstellung der erfindungsgemäßen Nanopartikeln aus einem hydrophilen Protein oder einer Kombination hydrophiler Proteine zur Überwindung der Blut-Hirn-Schranke umfaßt die folgenden Schritte:

- Desolvatieren einer wäßrigen Lösung eines hydrophilen Proteins oder einer Kombination hydrophiler Proteine,
- Stabilisieren der durch Desolvatation entstandenen Nanopartikel durch Quervernetzung,
- Umsetzen eines Teils der funktionellen Gruppen auf der Oberfläche der stabilisierten Nanopartikel zu reaktiven Thiolgruppen,
- kovalentes Anheften funktioneller Proteine mittels bifunktionaler Spacermoleküle,
- Biotinylierung von Apolipoprotein E, sofern die Partikel nicht kovalent gekoppeltes Apolipoprotein E aufweisen,
- Beladen der Avidin-modifizierten Nanopartikel mit biotinyliertem ApoE und dem zu verabreichenden pharmakologischen Wirkstoff.

Für die Herstellung der Nanopartikel wird ein hydrophiles Protein oder eine Kombination hydrophiler Proteine als Ausgangsmaterial verwendet. Vorzugsweise wird eine wäßrige Lösung von Serumalbumin, besonders bevorzugt von humanem Serumalbumin, unter Rühren desolvatisiert. Die entstehenden Nanopartikel werden durch Quervernetzung stabilisiert und die funktionellen Gruppen (Aminogruppen, Carboxylgruppen, Hydroxylgruppen) auf der Oberfläche der Nanopartikel werden durch geeignete Reagenzien zu reaktiven Thiolgruppen umgesetzt.

Die Desolvatation aus dem wäßrigen Lösungsmittel erfolgt vorzugsweise durch den Zusatz von Ethanol. Prinzipiell ist eine Desolvatation auch durch den Zusatz anderer wassermischbarer Nichtlösungsmittel für hydrophile Proteine wie Aceton, Isopropanol oder Methanol möglich. So wurde Gelatine als Ausgangsprotein erfolgreich durch Zugabe von Aceton desolvatisiert. Gleichfalls ist eine Desolvatation von in wäßriger Phase gelösten Proteinen durch Zugabe von strukturbildenden Salzen wie Magnesiumsulfat oder Ammoniumsulfat möglich. In diesem Fall spricht man von Aussalzen.

Als Quervernetzer zur Stabilisierung der Nanopartikel kommen bifunktionale Aldehyde, vorzugsweise Glutaraldehyd, sowie Formaldehyd in Frage. Ferner ist eine Quervernetzung der Nanopartikelmatrix durch thermische Prozesse möglich. Stabile Nanopartikelsysteme wurden bei 60°C über Zeiträume von mehr als 25 Stunden oder 70°C über Zeiträume von mehr als 2 Stunden erhalten.

Die Thiolierung der Nanopartikeloberfläche kann nach verschiedenen Prinzipien durchgeführt werden. Vorzugsweise werden die Aminogruppen auf der Partikeloberfläche mit 2-Iminothiolan, das mit primären Aminogruppen auf der Partikeloberfläche reagiert, zu freien Thiolgruppen auf der Partikeloberfläche umgesetzt. Daneben können Thiolgruppen auch durch reduktive Spaltung der an der Oberfläche der Nanopartikelmatrix vorhandenen Disulfid-Bindungen mit Dithiotreitol (DTT) erhalten werden. Alternativ können freie Carboxylgruppen der Partikeloberfläche mit 1-Ethyl-3-(3-dimethylaminopropyl)carbodiimid (EDC) / Cystein oder mit EDC / cystaminiumdichlorid umgesetzt und die so eingeführten Disulfid-Bindungen nachfolgend mit DTT reduktiv gespalten werden.

Funktionelle Proteine können über bifunktionale Spacermoleküle, die eine Reaktivität sowohl gegenüber Aminogruppen als auch gegenüber freien Thiolgruppen aufweisen, an die Thiolgruppen-modifizierten Nanopartikel gekoppelt werden. Anwendbar sind heterobifunktionale Spacermoleküle mit Reaktivitäten gegenüber Carboxyl- oder Hydroxylgruppen, aber auch homobifunktionale Spacermoleküle mit Reaktivitäten gegenüber Aminogruppen. Eine bevorzugte Substanz, die die Funktion eines bifunktionalen Spacermoleküls übernehmen kann, ist m-Maleimidobenzoyl-N-hydroxysulfosuccinimidoster (Sulfo-MBS). Neben m-Maleimidobenzoyl-N-hydroxysulfosuccinimidester wurden auch weitere heterobifunktionale Spacermoleküle wie Sulfosuccinimidyl-4-[N-maleimidomethyl]-cyclohexan-1-carboxylat (Sulfo-SMCC) oder Sulfosuccinimidyl-2-[m-azido-o-nitrobenzamido]-ethyl-1,3'-dithiopropionat (SAND) sowie die homobifunktionalen Spacermoleküle Dimethyl-3,3'-dithiobispropionimidat-dihydrochlorid (DTBP) oder 3,3'-Dithiobis[sulfo-succinimidylpropionat] (DTSSP) erfolgreich eingesetzt. Es werden jedoch heterobifunktionale Spacermoleküle bevorzugt, da homobifunktionale Spacermoleküle als Nebenreaktion zu der Anbindung eines funktionalen Proteins an die Nanopartikeloberfläche auch zu einer möglichen intramolekularen Quervernetzung führen.

In einem besonders bevorzugten Verfahren wird Avidin oder ein Avidin-Derivat mittels der bifunktionalen Spacermoleküle an die thiolierten Nanopartikel gekoppelt. Dieses Zwischenprodukt, Avidin-modifizierte Nanopartikel, stellt ein universelles Trägersystem für eine Vielzahl biotinylierter Substanzen dar, die über die Avidin-Biotin-Komplexbildung gebunden werden können.

Zur Bindung des humanen Apolipoproteins E an die Avidin-modifizierten Nanopartikel kann das Apolipoprotein E durch Umsetzung mit N-Hydroxysuccinimidobiotin (NHS-Biotin) biotinyliert werden. Andere Biotinylierungsreagenzien, die mit Aminogruppen oder anderen funktionellen Gruppen des zu bindenden Proteins reagieren, sind ebenfalls anwendbar. Als weitere funktionelle Gruppen des zu bindenden Proteins kommen für die Biotinylierung auch freie Sulfhydrylgruppen oder Carboxylgruppen in Frage. Alternative Biotinylierungsreagenzien für Aminogruppen unterscheiden sich von dem NHS-Biotin in der aminoreaktiven Funktionalität, indem sie z.B. Pentafluorphenyl-Gruppen anstelle von Succinimido-Gruppen haben, oder in dem Bereich zwischen Biotin und der aminoreaktiven Funktionalität.

Um pharmakologische Effekte zu vermitteln, werden pharmazeutisch oder biologisch aktive Substanzen in die Partikel eingearbeitet oder direkt bzw. indirekt an die Avidin-modifizierten Nanopartikel gebunden. Die Avidin-modifizierten Nanopartikel können gleichzeitig oder in beliebiger Reihenfolge mit biotinyliertem Apolipoprotein E und einem pharmazeutischen Wirkstoff beladen werden. Dabei kann die Bindung des Wirkstoffes sowohl kovalent, komplexierend über das Avidin-Biotin-System als auch adsorptiv erfolgen.

Die erfindungsgemäßen Nanopartikel aus einem hydrophilen Protein oder einer Kombination hydrophiler Proteine, die Apolipoprotein E gebunden haben, eignen sich, pharmazeutisch oder biologisch aktive Wirkstoffe, die sonst die Blut-Hirn-Schranke nicht passieren würden, insbesondere hydrophile Wirkstoffe, über die Blut-Hirn-Schranke zu transportieren und pharmakologische Effekte hervorzurufen. Beispiele für derartige Wirkstoffe sind Dalargin, Loperamid, Tubocuarin, Doxorubicin oder dergleichen.

Somit sind die beschriebenen wirkstoffbelandenen Nanopartikel zur Behandlung einer Vielzahl cerebraler Erkrankungen geeignet. Dabei werden die an das Trägersystem gebundenen Wirkstoffe nach dem jeweiligen Therapieziel ausgewählt. Das Trägersystem bietet sich vor allem für die Wirkstoffe an, die keinen oder keinen ausreichendem Übergang über die Blut-Hirn-Schranke aufweisen. Als Wirkstoffe kommen Zytostatika zur Therapie cerebraler Tumore in Betracht, Wirkstoffe zur Therapie viraler Infektionen im Cerebralbereich, beispielsweise HIV-Infektionen, aber auch Wirkstoffe zur Therapie von Demenz-Erkrankungen, um nur einige Anwendungsgebiete aufzuzählen.

FIG. 1 zeigt eine bevorzugte Ausführungsform der erfindungsgemäßen Nanopartikel ohne Wirkstoff oder weiteres funktionelles Protein.

Nachfolgend wird die Erfindung anhand einer beispielhaften Ausführungsform veranschaulicht, wobei diese Darstellung den Sinn und das Wesen der Erfindung in keiner Weise einschränkend aufgefasst werden soll.

Zur Herstellung von Nanopartikeln aus humanem Serumalbumin (HSA) wurden 200 mg humanes Serumalbumin in 2,0 ml gereinigtem Wasser gelöst. Zu dieser Lösung wurden unter Rühren auf einem Magnetrührer (500 rpm) 8,0 ml 96 Vol.-% Ethanol tropfenweise zugegeben.

Die erzeugten Nanopartikel wurden stabilisiert, indem dem Reaktionsansatz 235 $\mu$l einer wässrigen, 8%-igen (m/v) Glutaraldehydlösung zugegeben und über 24 Stunden bei Raumtemperatur gerührt wurde. Die stabilisierten Nanopartikel wurden durch fünfmaliges Abzentrifugieren (16.000 rcf, 8 min) und Redispergieren in 1,5 ml gereinigtem Wasser aufgereinigt. Durch gravimetrische Bestimmung wurde der resultierende Gehalt von Nanopartikeln in der Suspension festgestellt.

Anschließend wurden 2,0 ml der Nanopartikel-Suspension mit 2,0 ml einer Lösung von 13 mg 2 -Imiaothiolan (Traut's Reagenz) in Tris-Puffer (pH 8,5) versetzt und über 24 Stunden gerührt, um die Partikeloberfläche zu thiolieren. Die Nanopartikel wurden nach Thiolierung wie oben beschrieben aufgereinigt.

Das Avidin-Derivat NeutrAvidin™ wurde über m-Maleimidobenzoyl-N-hydroxysulfosuccinimidester (Sulfo-MBS), eine als bifunktionales Spacermolekül fungierende Substanz, kovalent an die thiolierten Nanopartikeln gebunden. Zu diesem Zweck wurde das Avidin-Derivat aktiviert, indem eine Lösung von 5,0 mg NeutrAvidin™ in 1,0 ml PBS-Puffer (pH 7,0) mit 1,6 mg Sulfo-MBS versetzt und über 1 Stunde bei Raumtemperatur gerührt wurde. Freies Sulfo-MBS wurde über eine Größenausschlußchromatographie von dem aktivierten NeutrAvidin abgetrennt.

Die Fraktionen, in denen durch spektrophotometrische Detektion bei 280 nm Wellenlänge NeutrAvidin nachgewiesen werden konnte, wurden vereinigt und mit 2,0 ml der thiolierten Nanopartikel versetzt und über 1 Stunde bei Raumtemperatur gerührt. Die Avidin-modifizierten HSA-Nanopartikel wurden wie oben beschrieben aufgereinigt.

Apolipoprotein E (ApoE) wurde biotinyliert, indem 250 $\mu$g ApoE in 125 $\mu$l isotonem PBS-Puffer, pH 7,4, gelöst wurden und diese Lösung mit einer Lösung von 150 $\mu$g NHS-Biotin (N-Hydroxysuccinimidobiotin) in 15 $\mu$l DMSO versetzt wurde. Nach einer Reaktionszeit von 2 Stunden bei 10°C unter Rühren wurde diese Mischung mit weiteren 300 $\mu$l PBS-Puffer, pH 7,4, verdünnt. Noch freies NHS-Biotin wurde über eine Größenausschlusschromatographie von dem biotinylierten ApoE abgetrennt. Die Fraktionen, in denen durch photometrische Detektion bei 280 nm Wellenlänge ApoE nachweisbar war, wurden vereinigt und gefriergetrocknet.

Die Avidin-modifizierten HSA-Nanopartikel wurden unmittelbar vor dem Tierversuch mit dem biotinylierten ApoE und dem Arz-. neistoff Dalargin beladen. Hierzu wurde das gefriergetrocknete ApoE in 250 $\mu$l destilliertem Wasser gelöst und mit 280 $\mu$l einer HSA-Nanopartikelsuspension, die 5,9 mg Avidin-modifizierte HSA-Nanopartikel enthielt, versetzt. Eine Lösung von 1,125 mg Dalargin in 470 $\mu$l Wasser wurde zugegeben und die Mischung wurde über 3 Stunden bei Raumtemperatur inkubiert. Nach dieser Inkubation wurde die Mischung durch Zugabe von 500 $\mu$l isotonem PBS-Puffer, pH 7,4, verdünnt.

Eine Quantifizierung der Beladung der Avidin-modifizierten HSA-Nanopartikel mit Dalargin ergab, dass bei einem Verhältnis von Dalargin / Nanopartikeln = 191 $\mu$g/mg eine adsorptive Bindung von 23,7 $\mu$g/mg (= 12,4%) Dalargin erfolgte.

Die applikationsfertige Zubereitung enthielt in einem Gesamtvolumen von 1,5 ml isotonem PBS-Puffer:

- 3,93 mg/ml Avidin-modifizierte HSA-Nanopartikel
- 167 $\mu$g/ml ApoE (über Avidin-Biotin-System an die Nanopartikel gebunden)
- 0,75 mg/ml Dalargin (davon 12,4 % adsorptiv an Nanopartikel gebunden).

Die Zubereitung wurde Mäusen i.v. in einer Dosierung von 7,5 mg/kg Dalargin appliziert. Das entspricht ausgehend von einem durchschnittlichen Körpergewicht einer Maus von 20 g, einer Applikationsmenge von 200 $\mu$l der oben aufgeführten Zubereitung je Maus.

Der analgetische Effekt (Nociceptive Response) wurde mit Hilfe des Tail-Flick-Tests ermittelt, bei dem ein heisser Lichtstrahl auf den Schwanz der Maus projiziert und die Zeit gemessen wird, bis die Maus den Schwanz wegzieht. Nach 10 Sekunden (= 100 % MPE) wurde das Experiment abgebrochen, um der Maus keinen Schaden zuzufügen. Der maximal mögliche analgetische Effekt (MPE = maximally possible effect) wurde gemäß der folgenden Formel ermittelt:

$$\% \text{ MPE} = \frac{\text{Reaktionszeit nach Applikation} - \text{Reaktionszeit vor Applikation}}{\text{Abbruchzeit} - \text{Reaktionszeit vor Applikation}} \times 100$$

Negative MPE-Werte ergaben sich, wenn die Maus ihren Schwanz nach Gabe der Zubereitung früher wegzieht als vor der Behandlung.

Mit Hilfe Dalargin-beladener Avidin-modifizierter HSA-Nanopartikel wurden nach intravenöser Injektion die in Tabelle 1 dargestellten analgetischen Effekte erreicht.

Tabelle 1

| Analgetischer Effekt [% MPE] bei Mäusen (n=6) nach i.v. Applikation von Dalargin (7,5 mg/kg) in Form einer der aufgeführten Präparationen. (MPE = Maximal Possible Effect) | | | | |
|---|---|---|---|---|
| Präparation | 30 min | 45 min | 90 min | 120 min |
| HSA-Avidin-Nanopartikel + ApoE + Dalargin | 25,1 $\pm$ 12,4 | 49,0 $\pm$ 23,7 | 2,1 $\pm$ 19,6 | -0,23 $\pm$ 12,3 |
| Kontrollen* | | | | |
| HSA-Avidin-Nanopartikel + Dalargin | -2,6 $\pm$ 3,9 | -5,4 $\pm$ 10,9 | -14,4 $\pm$ 17,4 | -9,6 $\pm$ 20,6 |
| PBCA-Nanopartikel + Dalargin + Polysorhat 80 | 35,2 $\pm$ 5,8 | 49,5 $\pm$ 4,5 | 36,5 $\pm$ 13,7 | 7,1 $\pm$ 6,3 |
| Dalargin-Lösung | 10,0 $\pm$ 9,8 | 9,3 $\pm$ 2,8 | 4,7 t 5,1 | 2,0 $\pm$ 6,1 |

*Die PBCA-Nanopartikel + Dalargin + Polysorbat 80 und die Dalargin-Lösungs-Vergleichsdaten stammen aus einem frühere Experiment.

Die Ergebnisse zeigen, dass die mit den Avidin-modifizierten HSA-Nanopartikeln erzielten analgetischen Effekte den Effekten entsprechen, die mit den Polybutylcyano-acrylat Nanopartikeln (PBCA-Nanopartikel) erreicht werden können.

**Patentansprüche**

1.  Nanopartikel zur Überwindung der Blut-Hirn-Schranke, **dadurch gekennzeichnet, dass** sie aus einem hydrophilen Protein oder einer Kombination hydrophiler Proteine bestehen, an die Apolipoprotein E gekoppelt oder gebunden ist.

2.  Nanopartikel nach Anspruch 1, **dadurch gekennzeichnet, dass** das mindestens ein hydrophiles Protein aus der Gruppe ausgewählt ist, die Serumalbumin, Gelatine A, Gelatine B, Casein und vergleichbare Proteine, oder eine Kombination dieser Proteine umfaßt.

3.  Nanopartikel nach Anspruch 1 oder 2, **dadurch gekennzeichnet, dass** mindestens ein hydrophiles Protein humanen Ursprungs ist.

4.  Nanopartikel nach einem der vorhergehenden Ansprüche, **dadurch gekennzeichnet, dass** sie ein oder mehrere unterschiedliche funktionelle Proteine aufweisen, die über bifunktionale Spacermoleküle an Thiolgruppen von Thiolgruppen-modifizierten Nanopartikeln gebunden sind.

5.  Nanopartikel nach Anspruch 4, **dadurch gekennzeichnet, dass** die funktionellen Proteine ausgewählt sind aus der Gruppe, die Avidin, Avidin-Derivate, Apolipoproteine, Antikörper, Enzyme, Hormone, Zytostatika umfaßt .

6.  Nanopartikel nach Anspruch 5, **dadurch gekennzeichnet, dass** biotinyliertes Apolipoprotein E über kovalent gekoppeltes Avidin gebunden ist.

7.  Nanopartikel nach Anspruch 6, **dadurch gekennzeichnet, dass** mindestens ein weiteres biotinyliertes funktionelles Protein über kovalent gekoppeltes Avidin gebunden ist.

8.  Nanopartikel nach einem der vorhergehenden Ansprüche, **dadurch gekennzeichnet, dass** sie pharmakologisch oder biologisch aktive Wirkstoffe inkorporiert oder gebunden haben.

9.  Nanopartikel nach Anspruch 8, **dadurch gekennzeichnet, dass** die pharmakologisch oder biologisch aktiven Wirkstoffe auf der Partikeloberfläche gebunden sind.

10. Nanopartikel nach Anspruch 8, **dadurch gekennzeichnet, dass** die pharmakologische oder biologisch aktiven Wirkstoffe kovalent, komplexierend über das Avidin-Biotin-System oder adsorptiv gebunden sind.

11. Nanopartikel nach einem der Ansprüche 8 bis 10, **dadurch gekennzeichnet, dass** die Wirkstoffe ausgewählt sind aus der Gruppe, die Dalargin, Loperamid, Tubocuarin und Doxorubicin umfaßt.

12. Verfahren zur Herstellung von Nanopartikeln aus einem hydrophilen Protein oder einer Kombination hydrophiler Proteine, zur Überwindung der Blut-Hirn-Schranke, **dadurch gekennzeichnet, dass** es die folgenden Schritte umfaßt:

    - Desolvatieren einer wäßrigen Lösung eines hydrophilen Proteins oder einer Kombination hydrophiler Proteine,
    - Stabilisieren der durch Desolvatation entstandenen Nanopartikel durch Quervernetzung,
    - Umsetzen eines Teils der funktionellen Gruppen auf der Oberfläche der stabilisierten Nanopartikel zu reaktiven Thiol-Gruppen,
    - kovalentes Anheften funktioneller Proteine, vorzugsweise von Avidin, mittels bifunktionaler Spacermoleküle,
    - Biotinylierung des Apolipoprotein E,
    - Beladen der Avidin-modifizierten Nanopartikel mit biotinyliertem Apolipoprotein E,
    - Beladen der Avidin-modifizierten Nanopartikel mit biotinyliertem Apolipoprotein E und weiteren funktionellen Proteinen oder pharmazeutisch oder biologisch aktiven Wirkstoffen.

13. Verfahren nach Anspruch 12, **dadurch gekennzeichnet, dass** das hydrophile Protein aus der Gruppe ausgewählt ist, die Serumalbumin, Gelatine A, Gelatine B, Casein und vergleichbare Proteine, oder eine Kombination dieser Proteine umfaßt .

14. Verfahren nach Anspruch 12 oder 13, **dadurch gekennzeichnet, dass** das hydrophile Protein humanen Ursprungs ist.

15. Verfahren nach einem der Ansprüche 12 bis 14, **dadurch gekennzeichnet, dass** das Desolvatieren durch Rühren und Zugabe eines wassermischbaren Nichtlösungsmittels für hydrophile Proteine oder durch Aussalzen erfolgt.

16. Verfahren nach Anspruch 15, **dadurch gekennzeichnet, dass** das wassermischbare Nichtlösungmittel für hydrophile Proteine aus der Gruppe ausgewählt wird, die Ethanol, Methanol, Isopropanol, und Aceton umfaßt.

17. Verfahren nach einem der Ansprüche 12 bis 16, **dadurch gekennzeichnet, dass** zum Stabilisieren der Nanopartikel thermische Prozesse oder bifunktionale Aldehyde oder Formaldehyd verwendet wird.

18. Verfahren nach Anspruch 17, **dadurch gekennzeichnet, dass** als bifuktionales Aldehyd Glutaraldehyd verwendet wird.

19. Verfahren nach einem der Ansprüche 12 bis 18, **dadurch gekennzeichnet, dass** als Thiolgruppen-modifizierendes Agens eine Substanz verwendet wird, die aus der Gruppe ausgewählt ist, die 2-Iminothiolan, eine Kombination aus 1-Ethyl-3-(3-dimethylaminopropyl)carbodiimid und Cystein oder eine Kombination aus 1-Ethyl-3-(3-dimethylaminopropyl)carbodiimid und Cystaminiumdichlorid sowie Dithiotreitol umfaßt.

20. verfahren nach einem der Ansprüche 12 bis 19, **dadurch gekennzeichnet, dass** als bifunktionales Spacermolekül eine Substanz verwendet wird, die aus der Gruppe ausgewählt ist, die m-Malelmu'dobenzoyl-N-hydroxysulfo-succinimidester, Sulfosuccinimidyl-4-[N-maleimido-methyl]cyclohexan-1-carboxylat, Sulfosuccinimidyl-2-[m-azido-o-nitrobenzamido]-ethyl-1,3'dithiopropionat, Dimethyl-3,3'-dithiobispropionimidatdihydrochlorid und 3,3'-Dithiobis [sulfosuccinimidylpropionat] umfaßt.

21. Verfahren nach einem der Ansprüche 12 bis 20, **dadurch gekennzeichnet, dass** die Wirkstoffe ausgewählt sind aus der Gruppe, die Dalargin, Loperamid, Tubocuarin und Doxorubicin umfaßt .

22. Nanopartikel, umfassend ein hydrophiles Protein oder eine Kombination hydrophiler Proteine, die Apolipoprotein E gebunden haben, für die Verwendung zum Transport pharmazeutisch oder biologisch aktiver Wirkstoff über die Blut-Hirn-Schranke.

23. Nanopartikel nach Anspruch 22, **dadurch gekennzeichnet, dass** mindestens eines der hydrophilen Proteine aus der Gruppe ausgewählt ist, die Serumalbumin, Gelatine A, Gelatine B, Casein und vergleichbare Proteine, oder eine Kombination dieser Proteine umfaßt.

24. Nanopartikel nach Anspruch 22 oder 23, **dadurch gekennzeichnet, dass** mindestens eines der hydrophilen Proteine humanen Ursprungs ist.

25. Nanopartikel nach einem der Ansprüche 22 bis 24, **dadurch gekennzeichnet, dass** die Wirkstoffe ausgewählt sind aus der Gruppe, die Dalargin, Loperamid, Tubocuarin und Doxorubicin umfaßt.

26. Nanopartikel nach einem der Ansprüche 22 bis 25 für die Verwendung in der Behandlung cerebraler Erkrankungen.


**Claims**

1. Nanoparticles for crossing the blood-brain barrier, **characterized in that** they consist of a hydrophilic protein or of a combination of hydrophilic proteins to which apolipoprotein E is coupled or bound.

2. Nanoparticles according to claim 1, **characterized in that** at least one hydrophilic protein is selected from the group comprising serum albumin, gelatine A, gelatine B, casein and comparable proteins, or comprises a combination of these proteins.

3. Nanoparticles according to claim 1 or 2, **characterized in that** at least one hydrophilic protein is of human origin.

4. Nanoparticles according to any one of the preceding claims, **characterized in that** they have one or more different functional proteins which are bound via bifunctional spacer molecules to thiol groups of thiol group-modified nanoparticles.

5. Nanoparticles according to claim 4, **characterized in that** the functional proteins are selected from the group comprising avidin, avidin derivatives, apolipoproteins, antibodies, enzymes, hormones, cytostatic agents.

6. Nanoparticles according to claim 5 **characterized in that** the biotinylated apolipoprotein E is bound via covalently coupled avidin.

7. Nanoparticles according to claim 6, **characterized in that** at least one further biotinylated functional protein is bound via covalently coupled avidin.

8. Nanoparticles according to any one of the preceding claims **characterized in that** they have incorporated therein or bound thereto pharmacologically or biologically active agents.

9. Nanoparticles according to claim 8, **characterized in that** the pharmacologically or biologically active agents are bound on the particle surface.

10. Nanoparticles according to claim 8, **characterized in that** the pharmacologically or biologically active agents are bound covalently, or by complex formation via the avidin-biotin system, or adsorptively.

11. Nanoparticles according to any one of claims 8 to 10, **characterized in that** the active agents are selected from the group comprising dalargin, loperamide, tubocurarine and doxorubicin.

12. Process for the manufacture of nanoparticles of one hydrophilic protein or a combination of hydrophilic proteins for crossing the blood-brain barrier, **characterized in that** it comprises the following steps:

    - desolvating an aqueous solution of a hydrophilic protein or a combination of hydrophilic proteins,
    - stabilising the nanoparticles produced by the desolvation by crosslinking,
    - converting a part of the functional groups on the surface of the stabilised nanoparticles to reactive thiol groups,
    - covalently attaching functional proteins, preferably avidin, by means of bifunctional spacer moleo-Liles,
    - biotinylating the apolipoprotein E,
    - loading the avidin-modified nanoparticles with biotinylated apolipoprotein E
    - loading the avidin-modified nanoparticles with biotinylated apolipoprotein E and with further functional proteins or pharmaceutically or biologically active substances.

13. Process according to claim 12, **characterized in that** the hydrophilic protein is selected from the group comprising serum albumin, gelatine A, gelatine B, casein and comparable proteins, or comprises a combination of these proteins.

14. Process according to claim 12 or 13, **characterized in that** the hydrophilic protein is of human origin.

15. Process according to claims 12 to 14, **characterized in that** the desolvation is performed by stirring and adding a water-miscible non-solvent for hydrophilic proteins or by salting-out.

16. Process according to claim 15, **characterized in that** the water-miscible non-solvent for hydrophilic proteins is selected from the group comprising ethanol, methanol, isopropanol and acetone.

17. Process according to any one of claims 12 to 16, **characterized in that** for stabilizing the nanoparticles, thermal processes or bifunctional aldehydes or formaldehyde is used.

18. Process according to claim 17, **characterized in that** glutaraldehyde is used as bifunctional aldehyde.

19. Process according to any one of claims 12 to 18, **characterized in that** as thiol group-modified agent a substance is used which is selected from the group comprising 2-iminothiolane, a combination of 1-ethyl-3- (3-dimethylaminopropyl) carbodiimide and cysteine, or a combination of 1-ethyl-3-(3-dimethylaminopropyl)carbodiimide and cystaminium dichloride as well as dithiothreitol.

20. Process according to any one of claims 12 to 19, **characterized in that** as bifunctional spacer molecule a substance is used which is selected from the group comprising m-maleimidobenzoyl-N-hydroxysulfosuccinimide ester, sulfosuccinimidyl-4-[N-maleimidomethyl]-cyclohexane-1-carboxylate, sulfosuccinimidyl-2-[m-azido-o-nitrobenzamido]-ethyl-1,3'-dithiopropionate, dimethyl-3,-3'-dithiobispropionimidate-dihydrochloride and 3,3'-dithio-bis[sulfosuccinimidylpropionate].

21. Process according to any one of claims 12 to 20, **characterized in that** the active substances are selected from the group comprising dalargin, loperamide, tubocurarine and doxorubicin.

22. Nanoparticles comprising a hydrophilic protein or a combination of hydrophilic proteins which have bound thereto apolipoprotein E, for use in the transport of pharmaceutically or biologically active agents across the blood-brain barrier.

23. Nanoparticles according to claim 22, **characterized in that** at least one of the hydrophilic proteins is selected from the group comprising serum albumin, gelatine A, gelatine B, casein and comparable proteins, or comprises a combination of these proteins.

24. Nanoparticles according to claim 22 or 23, **characterized in that** at least one of the hydrophilic proteins is of human origin.

25. Nanoparticles according to any one of claims 22 to 24, **characterized in that** the active agents are selected from the group comprising dalargin, loperamide, tubocurarine and doxorubicin.

26. Nanoparticles according to any one of claims 22 to 25, for use in the treatment of cerebral affections.


**Revendications**

1. Nanoparticules servant à traverser la barrière sang-cerveau, **caractérisées en ce qu'**elles sont constituées d'une protéine hydrophile ou d'une combinaison de protéines hydrophiles, protéines auxquelles de l'apolipoprotéine E est couplée ou liée.

2. Nanoparticules selon la revendication 1, **caractérisées en ce que** la au moins une protéine hydrophile est sélectionnée dans le groupe qui contient de l'albumine de sérum, de la gélatine A, de la gélatine B, de la caséine et des protéines comparables, ou une combinaison de ces protéines.

3. Nanoparticules selon la revendication 1 ou 2, **caractérisées en ce qu'**au moins une protéine hydrophile est d'origine humaine.

4. Nanoparticules selon l'une quelconque des revendications précédentes, **caractérisées en ce qu'**elles présentent une ou plusieurs protéines fonctionnelles différentes, qui sont liées par des molécules d'espaceur (spacer) bifonctionnelles à des groupes thiol de nanoparticules modifiées par des groupes thiol.

5. Nanoparticules selon la revendication 4, **caractérisées en ce que** les protéines fonctionnelles sont sélectionnées dans le groupe qui comprend de l'avidine, des dérivés d'avidine, des apolipoprotéines, des anticorps, des enzymes, des hormones, des cytostatiques.

6. Nanoparticules selon la revendication 5, **caractérisées en ce que** de l'apolipoprotéine E biotinylée est liée par de l'avidine couplée de façon covalente.

7. Nanoparticules selon la revendication 6, **caractérisées en ce qu'**au moins une autre protéine fonctionnelle biotinylée est liée par de l'avidine couplée de façon covalente.

8. Nanoparticules selon l'une quelconque des revendications précédentes, **caractérisées en ce qu'**elles ont incorporé ou lié des agents actifs au plan pharmacologique ou biologique.

9. Nanoparticules selon la revendication 8, **caractérisées en ce que** les agents actifs au plan pharmacologique ou biologique sont liés sur la surface de particule.

**10.** Nanoparticules selon la revendication 8, **caractérisées en ce que** les agents actifs au plan pharmacologique ou biologique sont liés de façon covalente, complexante par le système avidine-biotine ou par adsorption.

**11.** Nanoparticules selon l'une quelconque des revendications 8 à 10, **caractérisées en ce que** les agents actifs sont sélectionnés dans le groupe qui comprend les éléments suivants : dalargine, lopéramide, tubocuarine et doxorubicine.

**12.** Procédé de préparation de nanoparticules à partir d'une protéine hydrophile ou d'une combinaison de protéines hydrophiles, destinées à traverser la barrière sang-cerveau, **caractérisé en ce qu'**il comprend les étapes suivantes :

- désolvatation d'une solution aqueuse d'une protéine hydrophile ou d'une combinaison de protéines hydrophiles,
- stabilisation des nanoparticules formées par désolvatation par réticulation transversale,
- transformation d'une partie des groupes fonctionnels à la surface des nanoparticules stabilisées en groupes thiol réactifs,
- adhérence covalente de protéines fonctionnelles, de préférence d'avidine, au moyen de molécules d'espaceur bifonctionnelles,
- biotinylation de l'apolipoprotéine E,
- chargement des nanoparticules modifiées par avidine avec de l'apolipoprotéine E biotinylée,
- chargement des nanoparticules modifiées par avidine avec de l'apolipoprotéine E biotinylée et d'autres protéines fonctionnelles ou des agents actifs au plan pharmaceutique ou biologique.

**13.** Procédé selon la revendication 12, **caractérisé en ce que** la protéine hydrophile est sélectionnée dans le groupe suivant : albumine de sérum, gélatine A, gélatine B, caséine et protéines comparables, ou une combinaison de ces protéines.

**14.** Procédé selon la revendication 12 ou 13, **caractérisé en ce que** la protéine hydrophile est d'origine humaine.

**15.** Procédé selon l'une quelconque des revendications 12 à 14, **caractérisé en ce que** la désolvatation s'effectue par agitation et addition d'un non-solvant miscible à l'eau pour des protéines hydrophiles ou par précipitation saline.

**16.** Procédé selon la revendication 15, **caractérisé en ce que** le non-solvant miscible à l'eau pour des protéines hydrophiles est sélectionné dans le groupe qui comprend les éléments suivants : éthanol, méthanol, isopropanol et acétone.

**17.** Procédé selon l'une quelconque des revendications 12 à 16, **caractérisé en ce que** des processus thermiques ou des aldéhydes bifonctionnels ou du formaldéhyde sont utilisés pour la stabilisation des nanoparticules,

**18.** Procédé selon la revendication 17, **caractérisé en ce que** du glutaraldéhyde est utilisé comme aldéhyde bifonctionnel.

**19.** Procédé selon l'une quelconque des revendications 12 à 18, **caractérisé en ce qu'**on utilise comme agent de modification de groupes thiol une substance qui est sélectionnée dans le groupe qui comprend les éléments suivants v 2-iminothiolane, une combinaison de 1-éthyl-3-(3-diméthylaminopropyl)carbodiimide et de cystéine ou une combinaison de 1-éthyl-3-(3-diméthylaminopropyl)carbodiimide et du dichlorure de cystaminium ainsi que du dithiothréitol.

**20.** Procédé selon l'une quelconque des revendications 12 à 19, **caractérisé en ce qu'**on utilise comme molécule d'espaceur bifonctionnelle une substance qui est sélectionnée dans le groupe qui comprend les éléments suivants :

ester m-maléimidobenzoyl-N-hydroxysulfo- succinimidique, sulfosuccinimidyl-4-[N-maléimido-méthyl]cyclohexane-1-carboxylate, sulfosuccinimidyl-2-[m-azido-o-nitrobenzamido]-éthyl-1,3'-dithiopropionate, dihydrochlorure de diméthyl-3,3'-dithiobispropionimidate et 3,3'-dithiobis[sulfosuccinimidyl propionate].

**21.** Procédé selon l'une quelconque des revendications 12 à 20, **caractérisé en ce que** les agents actifs sont sélectionnés dans le groupe qui comprend les éléments suivants: dalargine, lopéramide, tubocuarine et doxorubicine.

**22.** Nanoparticules comprenant une protéine hydrophile ou une combinaison de protéines hydrophiles, qui ont lié de l'apolipoprotéine E, destinée à l'utilisation pour le transport d'agents actifs au plan pharmaceutique ou biologique à travers de la barrière sang-cerveau.

23. Nanoparticules selon la revendication 22, **caractérisées en ce qu'**au moins l'une des protéines hydrophiles est sélectionnée dans le groupe qui comprend les éléments suivants : albumine de sérum, gélatine A, gélatine B, caséine et des protéines comparables, ou une combinaison de ces protéines.

24. Nanoparticules selon la revendication 22 ou 23, **caractérisées en ce qu'**au moins l'une des protéines hydrophiles est d'origine humaine.

25. Nanoparticules selon l'une quelconque des revendications 22 à 24, **caractérisées en ce que** les agents actifs sont sélectionnés dans le groupe qui comprend les éléments suivants: dalargine, lopéramide, tubocuarine et doxotubicine.

26. Nanoparticules selon l'une quelconque des revendications 22 à 25 pour une utilisation dans le traitement de maladies cérébrales.

COOH

OH  Thiol-Modifikation  bifunkt. Spacer  Avidin

—NH₂

—COOH

Nanopartikel Matrix

biotinyliertes
Apolipoprotein

FIG. 1